# EUROPÄISCHE PATENTANMELDUNG

(11) **EP 0 705 820 A1**
(43) Veröffentlichungstag der Anmeldung: **10.04.1996**
(21) Anmeldenummer: 95112833.9
(22) Anmeldetag: 16.08.1995
(51) Int. Cl.: C07D 211/92, C07D 211/90, A61K 31/44, C07D 471/02, C07D 213/80

(54) **6-Amino-Nicotinsäurederivaten und ihre Verwendung als selektive Kaliumkanalmodulatoren**

(30) Priorität: 29.08.1994 DE 4430638
(71) Anmelder: BAYER AG, D-51368 Leverkusen (DE)
(72) Erfinder: Urbahns, Klaus, Dr., D-42115 Wuppertal (DE); Goldmann, Siegfried, Dr., D-42327 Wuppertal (DE); Heine, Hans-Georg, Dr., D-47800 Krefeld (DE); Junge, Bodo, Dr., D-42399 Wuppertal (DE); Schohe-Loop, Rudolf, Dr., D-42327 Wuppertal (DE); Sommermeyer, Henning, Dr., D-51061 Köln (DE); Glaser, Thomas, Dr., D-51491 Overath (DE); Wittka, Reilinde, Dr., D-51069 Köln (DE); de Vry, Jean-Marie-Viktor, Dr., D-51503 Rösrath (DE)

(57) **Zusammenfassung**

Die Anmeldung betrifft substituierte 4-Phenyl-6-amino-nicotinsäurederivate zur therapeutischen Anwendung, neue Wirkstoffe sowie deren Verwendung als cerebral wirksame Mittel. Die Wirkstoffe werden hergestellt, indem man entsprechend substituierte Dihydropyridine nach üblichen Methoden oxidiert.

## Beschreibung

Die vorliegende Erfindung betrifft teilweise bekannte substituierte 4-Phenyl-6-amino-nicotinsäurederivate als Arzneimittel, neue Wirkstoffe, ein Verfahren zu deren Herstellung, und ihre Verwendung als Kaliumkanalmodulatoren, insbesondere zur Behandlung des zentralen Nervensystems.

Aus den Publikationen [Collect. Czech. Chem. Comun. 56 (10), 2175-82, 1991 und Khim. Geterotsikl. Soedin., (11), 1504 - 8, 1984] sind einige 4-Phenyl-3-pyridin-carbonsäurederivate bekannt, wobei dort keine pharmakologische Wirkung beschrieben wird.

Es wurde nun gefunden, daß die teilweise bekannten substituierten 4-Phenyl-6-amino-nicotinsäurederivate der allgemeinen Formel (I), 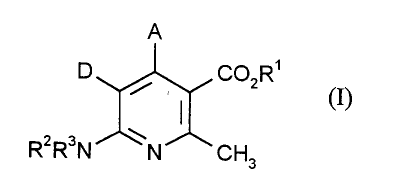
in welcher
- A: für Aryl mit 6 bis 10 Kohlenstoffatomen oder Pyridyl steht, die gegebenenfalls bis zu 3-fach gleich oder verschieden durch Nitro, Cyano, Phenyl, Halogen, Trifluormethyl oder durch geradkettiges oder verzweigtes Alkylthio oder Alkoxy mit jeweils bis zu 6 Kohlenstoffatomen substituiert sind,
- D: für Cyano oder Nitro steht,
- R¹: für Wasserstoff oder für geradkettiges oder verzweigtes Alkyl mit bis zu 8 Kohlenstoffatomen steht,
- R² und R³: gleich oder verschieden sind und
für Wasserstoff oder für geradkettiges oder verzweigtes Alkyl oder Acyl mit jeweils bis zu 6 Kohlenstoffatomen stehen
und deren Salze
überraschenderweise eine modulierende Wirkung auf Kaliumkanäle besitzen und somit geeignet sind zur Verwendung bei der Bekämpfung von cerebralen Erkrankungen und der Sichelzellenanämie.

Im Rahmen der Erfindung sind physiologisch verträgliche Salze bevorzugt. Physiologisch unbedenkliche Salze sind im allgemeinen Salze der erfindungsgemäßen Verbindungen mit anorganischen oder organischen Säuren. Bevorzugt werden Salze mit anorganischen Säuren wie beispielsweise Salzsäure, Bromwasserstoffsäure, Phosphorsäure oder Schwefelsäure, oder Salze mit organischen Carbon- oder Sulfonsäuren wie beispielsweise Essigsäure, Maleinsäure, Fumarsäure, Äpfelsäure, Zitronensäure, Weinsäure, Milchsäure, Benzoesäure, oder Methansulfonsäure, Ethansulfonsäure, Phenylsulfonsäure, Toluolsulfonsäure oder Naphthalindisulfonsäure.

Die erfindungsgemäßen Verbindungen können in stereoisomeren Formen existieren, die sich entweder wie Bild und Spiegelbild (Enantiomere), oder die sich nicht wie Bild und Spiegelbild (Diastereomere) verhalten. Die Erfindung betrifft sowohl die Antipoden als auch die Racemformen sowie die Diastereomerengemische. Die Racemformen lassen sich ebenso wie die Diastereomeren in bekannter Weise in die stereoisomer einheitlichen Bestandteile trennen.

Bevorzugt sind Verbindungen der allgemeinen Formel (I),
in welcher
- A: für Phenyl oder Naphthyl steht, die gegebenenfalls bis zu 3-fach gleich oder verschieden durch Nitro, Cyano, Fluor, Chlor, Brom, Iod, Phenyl, Trifluormethyl oder durch geradkettiges oder verzweigtes Alkylthio oder Alkoxy mit jeweils bis zu 4 Kohlenstoffatomen substituiert sind,
- D: für Cyano oder Nitro steht,
- R¹: für Wasserstoff oder für geradkettiges oder verzweigtes Alkyl mit bis zu 6 Kohlenstoffatomen steht,
- R² und R³: gleich oder verschieden sind und für Wasserstoff oder für geradkettiges oder verzweigtes Alkyl oder Acyl mit jeweils bis zu 4 Kohlenstoffatomen stehen
und deren Salze,
bei der Bekämpfung von cerebralen Erkrankungen.
Besonders bevorzugt sind Verbindungen der allgemeinen Formel (I),
in welcher

- A: für Phenyl steht, das gegebenenfalls bis zu 2-fach gleich oder verschieden durch Nitro, Cyano, Fluor, Chlor, Brom, Iod, Phenyl, Trifluormethyl, Methoxy oder Methylthio substituiert ist,
- D: für Cyano oder Nitro steht,
- R¹: für Wasserstoff oder für geradkettiges oder verzweigtes Alkyl mit bis zu 4 Kohlenstoffatomen steht,
- R² und R³: gleich oder verschieden sind und für Wasserstoff oder für geradkettiges oder verzweigtes Alkyl oder Acyl mit jeweils bis zu 3 Kohlenstoffatomen stehen
und deren Salze
bei der Bekämpfung von cerebralen Erkrankungen.

Die erfindungsgemäßen Verbindungen der allgemeinen Formel (I) zeigen ein nicht vorhersehbares, wertvolles pharmakologisches Wirkspektrum.

Sie sind Kanalmodulatoren mit Selektivität für Calciumabhängige Kalium-Kanäle großer Leitfähigkeit (BK(Ca)-Kanäle), insbesondere des zentralen Nervensystems.

Aufgrund dieser pharmakologischen Eigenschaften sind sie für die Herstellung von Arzneimitteln zur Behandlung von zentral degenerativen Erkrankungen geeignet, wie z.B. bei Auftreten von Demenzen: Multiinfarktdemenz (MID), primär degenerativer Demenz (PDD), präseniler und seniler Alzheimerscher Krankheit, HIV-Demenz und andere Demenzformen, außerdem zur Behandlung der Parkinsonschen Krankheit oder amyotrophischen Lateralsklerose sowie multiplen Sklerose.

Weiterhin eignen sich die Wirkstoffe zur Behandlung von Hirnleistungsstörungen im Alter, des hirnorganischen Psychosyndroms (HOPS, Organic Brain Syndrom, OBS) und von altersbedingten Gedächtnisstörungen (age associated memory impairment, AAMI).

Sie sind geeignet zur Prophylaxe, Behandlung, und zur Bekämpfung der Folgen cerebraler Durchblutungsstörungen wie cerebraler Ischämien, Schlaganfällen, Schädel-Hirn-Traumata und von Subarachnoidalblutungen.

Sie sind wertvoll zur Behandlung von Depressionen und Psychosen, z.B. Schizophrenie. Außerdem eignen sie sich zur Behandlung von Störungen der neuroendokrinen Sekretion sowie der Neurotransmittersekretion und damit zusammenhängenden gesundheitlichen Störungen wie Manie, Alkoholismus, Drogenmißbrauch, Sucht oder krankhaftem Eßverhalten. Weitere Anwendungsgebiete sind die Behandlung von Migräne, Schlafstörungen und von Neuropathien. Darüber hinaus sind sie als Schmerzmittel geeignet.

Die Wirkstoffe sind ferner geeignet zur Behandlung von Störungen des Immunsystems, insbesondere der T-Lymphocyten-Proliferation und zur Beeinflussung der glatten Muskulatur, insbesondere von Uterus, Harnblase und Bronchialtrakt und zur Behandlung damit zusammenhängender Krankheiten wie z.B. Asthma und urinärer Inkontinenz und zur Behandlung von Bluthochdruck, Arrhythmie, Angina und Diabetes.

Die Erfindung betrifft auch neue Stoffe der allgemeinen Formel (I),
in welcher
A, D, R¹ bis R³ die angegebene Bedeutung haben,
wobei jedoch folgende Verbindungen ausgenommen sind:
6-Amino-5-cyano-2-methyl-4-phenyl-nicotinsäureethylester
6-Amino-4-(4-chlorphenyl)-5-cyano-2-methyl-nicotinsäureethylester
6-Amino-5-cyano-2-methyl-4-(4-methoxyphenyl)-nicotinsäureethylester
6-Amino-5-cyano-2-methyl-4-(4-nitrophenyl)-nicotinsäureethylester.

Die Erfindung betrifft bevorzugt die neue Verbindungen der allgemeinen Formel (Ia) 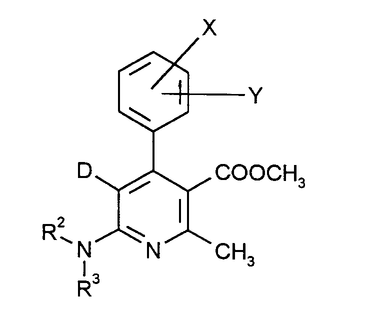
und deren Salze,
mit den in der folgenden Tabelle angegebenen Substituentenbedeutungen:

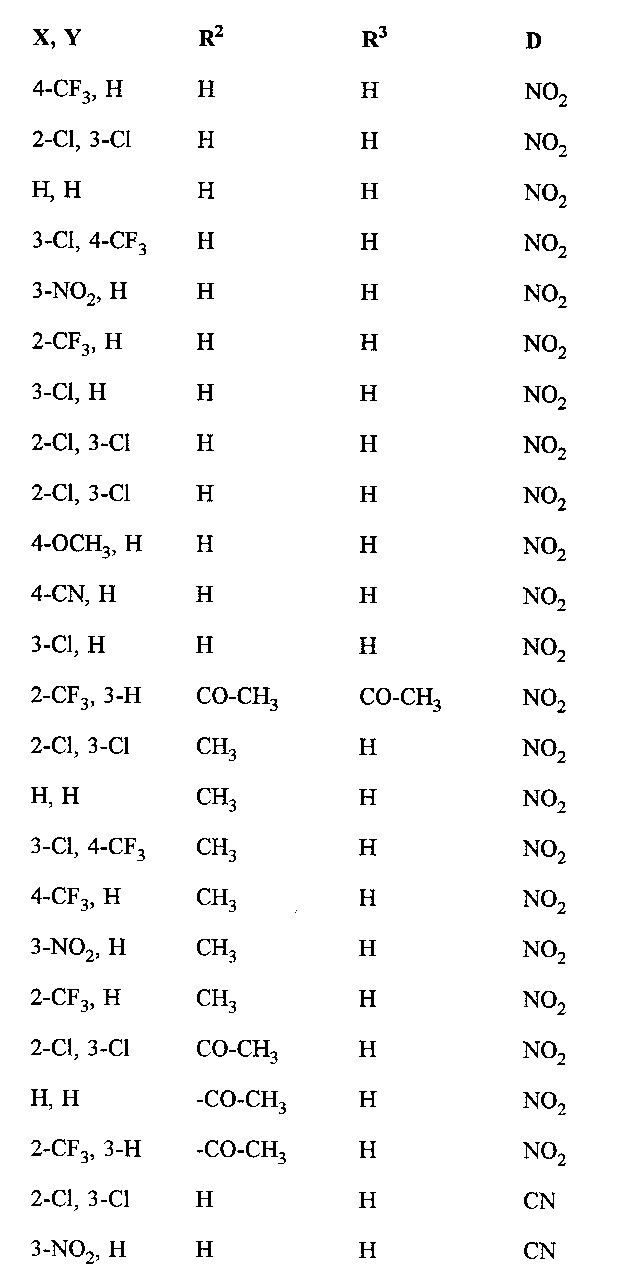

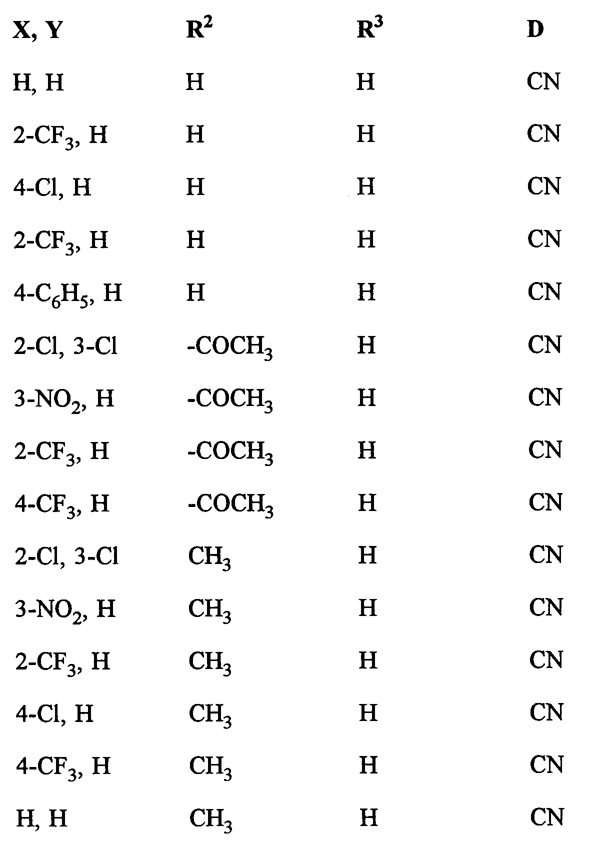

Die neuen Verbindungen der allgemeinen Formel (I) werden hergestellt, indem man
Dihydropyridine der allgemeinen Formel (II) 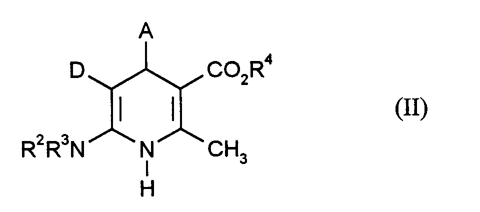
in welcher
- A, D, R¹ bis R³: die angegebene Bedeutung haben,
- R⁴: die oben angegebene Bedeutung von R¹ und R^{1'} hat, aber nicht für Wasserstoff steht,
in einem inerten Lösemittel mit einem typischen Oxidationsmittel, vorzugsweise Mangandioxid, oxidiert,
die Produkte gegebenenfalls in organischen Lösemitteln und in Anwesenheit einer Base alkyliert oder acyliert
und gegebenenfalls die Ester hydrolysiert.

Das erfindungsgemäße Verfahren kann durch folgendes Formelschema beispielhaft erläutert werden: 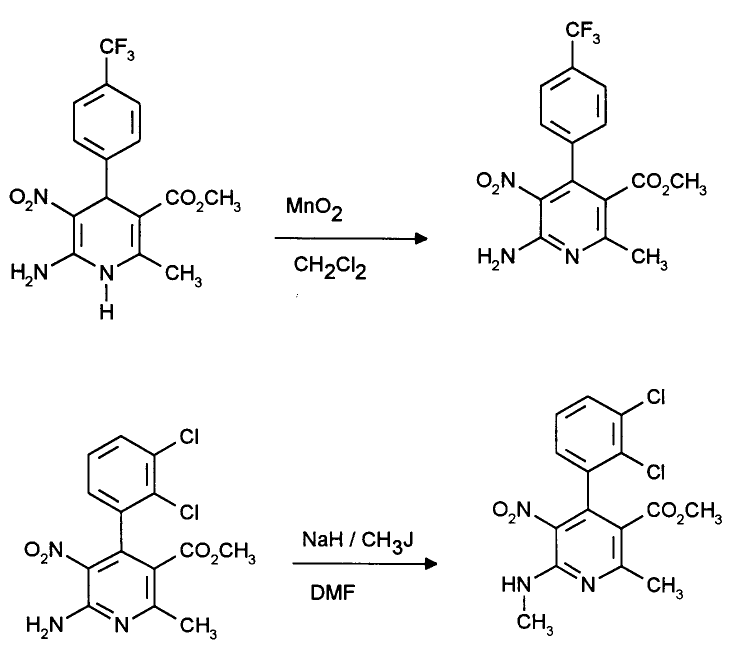
Als Lösemittel eignen sich hierbei alle inerten organischen Lösemittel, die sich unter den Reaktionsbedingungen nicht verändern. Hierzu gehören bevorzugt Alkohole wie Methanol, Ethanol, Propanol oder Isopropanol, oder Ether wie Diethylether, Dioxan, Tetrahydrofuran, Glykoldimethylether, oder Diethylenglykoldimethylether, Acetonitril, oder Amide wie Hexamethylphosphorsäuretriamid oder Dimethylformamid, oder Essigsäure oder halogenierte Kohlenwasserstoffe wie Methylenchlorid, Tetrachlorkohlenstoff oder Kohlenwasserstoffe wie Benzol oder Toluol. Ebenso ist es möglich, Gemische der genannten Lösemittel zu verwenden. Besonders bevorzugt ist Methylenchlorid.

Als Lösemittel für die Oxidation eignen sich alle inerten organischen Lösemittel, die sich unter den Reaktionsbedingungen nicht verändern. Hierzu gehören bevorzugt Alkohole wie Methanol, Ethanol, Propanol oder Isopropanol, oder Ether wie Diethylether, Dioxan, Tetrahydrofuran, Glykoldimethylether, oder Diethylenglykoldimethylether, Acetonitril, oder Amide wie Hexamethylphosphorsäuretriamid oder Dimethylformamid, oder Essigsäure oder halogenierte Kohlenwasserstoffe wie Methylenchlorid, Tetrachlorkohlenstoff oder Kohlenwasserstoffe wie Benzol oder Toluol. Ebenso ist es möglich, Gemische der genannten Lösemittel zu verwenden. Bevorzugt ist Methylenchlorid.

Als Oxidationsmittel eignen sich im allgemeinen 2,3-Dichlor-4,5-dicyan-p-benzochinon und Derivate, Pyridiniumdichromat, elementares Brom, Jod und Mangandioxid. Bevorzugt ist Mangandioxid.

Das Oxidationsmittel wird im allgemeinen in einer Menge von 1 mol bis 20 mol, bevorzugt von 1 mol bis 5 mol, bezogen auf 1 mol der Verbindungen der allgemeinen Formel (II) eingesetzt.

Die Reaktionstemperaturen können in einem größeren Bereich variiert werden. Im allgemeinen arbeitet man zwischen +10°C und +150°C, vorzugsweise zwischen +20°C und +100°C, insbesondere bei Raumtemperatur.

Die Umsetzungen können bei Normaldruck, aber auch bei erhöhtem oder erniedrigtem Druck (z.B. 0,5 bis 3 bar) durchgeführt werden. Im allgemeinen arbeitet man bei Normaldruck.

Als Lösemittel für die Alkylierung eignen sich ebenfalls übliche organische Lösemittel, die sich unter den Reaktionsbedingungen nicht verändern. Hierzu gehören bevorzugt Ether wie Diethylether, Dioxan, Tetrahydrofuran, Glykoldimethylether, oder Kohlenwasserstoffe wie Benzol, Toluol, Xylol, Hexan, Cyclohexan oder Erdölfraktionen, oder Halogenkohlenwasserstoffe wie Dichlormethan, Trichlormethan, Tetrachlormethan, Dichlorethylen, Trichlorethylen oder Chlorbenzol, oder Essigester, oder Triethylamin, Pyridin, Dimethylsulfoxid, Dimethylformamid, Hexamethylphosphorsäuretriamid, Acetonitril, Aceton oder Nitromethan. Ebenso ist es möglich, Gemische der genannten Lösemittel zu verwenden. Bevorzugt ist Dimethylformamid.

Als Basen eignen sich im allgemeinen Alkalihydride- oder alkoholate, wie beispielsweise Natriumhydrid oder Kalium-tert.butylat, oder cyclische Amine, wie beispielsweise Piperidin, Dimethylaminopyridin oder C₁-C₄-Alkylamine, wie beispielsweise Triethylamin. Bevorzugt ist Natriumhydrid.

Die Reaktionstemperaturen können in einem größeren Bereich variiert werden. Im allgemeinen arbeitet man zwischen +10°C und +150°C, vorzugsweise zwischen +20°C und +100°C, insbesondere bei Raumtemperatur.

Die Alkylierung wird in den oben aufgeführten Lösemitteln bei Temperaturen von 0°C bis +150° C, vorzugsweise bei Raumtemperaturen bis +100°C durchgeführt.

Die Umsetzungen können bei Normaldruck, aber auch bei erhöhtem oder erniedrigtem Druck (z.B. 0,5 bis 3 bar) durchgeführt werden. Im allgemeinen arbeitet man bei Normaldruck.

Die Base wird im allgemeinen in einer Menge von 1 mol bis 5 mol, bevorzugt von 1 mol bis 2 mol jeweils bezogen auf 1 mol der zu alkylierenden Verbindungen eingesetzt.

Als Basen für die Acylierung eignen sich anorganische oder organische Basen. Hierzu gehören vorzugsweise Alkalihydroxide wie z.B. Natriumhydroxid oder Kaliumhydroxid, Erdalkalihydroxide wie z.B. Bariumhydroxid, Alkalicarbonate wie Natriumcarbonat oder Kaliumcarbonat, Erdalkalicarbonate wie Calciumcarbonat, oder organische Amine Trialkyl(C₁-C₆)amine wie Triethylamin, oder Heterocyclen wie Pyridin, Methylpiperidin, Piperidin oder Morpholin. Besonders bevorzugt ist Triethylamin.

Als Lösemittel für die Acylierung eignen sich ebenfalls übliche organische Lösemittel, die sich unter den Reaktionsbedingungen nicht verändern. Hierzu gehören bevorzugt Ether wie Diethylether, Dioxan, Tetrahydrofuran, Glykoldimethylether, oder Kohlenwasserstoffe wie Benzol, Toluol, Xylol, Hexan, Cyclohexan oder Erdölfraktionen, oder Halogenkohlenwasserstoffe wie Dichlormethan, Trichlormethan, Tetrachlormethan, Dichlorethylen, Trichlorethylen oder Chlorbenzol, oder Essigester, oder Triethylamin, Pyridin, Dimethylsulfoxid, Dimethylformamid, Hexamethylphosphorsäuretriamid, Acetonitril, Aceton oder Nitromethan. Ebenso ist es möglich, Gemische der genannten Lösemittel zu verwenden oder das jeweilige Acylierungsmittel auch als Lösemittel einzusetzen. Bevorzugt sind Acetanhydrid und Pyridin

Die Acylierung verläuft im allgemeinen in einem Temperaturbereich von 0°C bis +120°C, vorzugsweise bei +30°C bis +90°C und bei Normaldruck.

Die Verseifung der Carbonsäureester erfolgt nach üblichen Methoden, indem man die Ester in inerten Lösemitteln mit üblichen Basen behandelt.

Als Basen eignen sich für die Verseifung die üblichen anorganischen Basen. Hierzu gehören bevorzugt Alkalihydroxide oder Erdalkalihydroxide wie beispielsweise Natriumhydroxid, Kaliumhydroxid oder Bariumhydroxid, oder Alkalicarbonate wie Natrium- oder Kaliumcarbonat oder Natriumhydrogencarbonat. Besonders bevorzugt werden Natriumhydroxid oder Kaliumhydroxid eingesetzt.

Als Lösemittel eignen sich für die Verseifung Wasser oder die für eine Verseifung üblichen organischen Lösemittel. Hierzu gehören bevorzugt Alkohole wie Methanol, Ethanol, Propanol, Isopropanol oder Butanol, oder Ether wie Tetrahydrofuran oder Dioxan, oder Dimethylformamid oder Dimethylsulfoxid. Besonders bevorzugt werden Alkohole wie Methanol, Ethanol, Propanol oder Isopropanol verwendet. Ebenso ist es möglich, Gemische der genannten Lösemittel einzusetzen.

Die Verseifung wird im allgemeinen in einem Temperaturbereich von 0°C bis +100°C, bevorzugt von +20°C bis +80°C durchgeführt.

Im allgemeinen wird die Verseifung bei Normaldruck durchgeführt. Es ist aber auch möglich, bei Unterdruck oder bei Überdruck zu arbeiten (z.B. von 0,5 bis 5 bar).

Enantiomerenreine Formen erhält man z.B., indem man Diastereomerengemische der Verbindungen der allgemeinen Formeln (I) und (Ia), in welcher R¹ / R^{1'} für einen optisch aktiven Esterrest steht, nach üblicher Methode trennt, anschließend entweder direkt umestert oder zuerst die chiralen Carbonsäuren herstellt und dann durch Veresterung die enantiomerenreinen Verbindungen herstellt.

Die Trennung der Diastereomeren erfolgt im allgemeinen entweder durch fraktionierte Kristallisation, durch Säulenchromatographie oder durch Craig-Verteilung. Welches das optimale Verfahren ist, muß von Fall zu Fall entschieden werden, manchmal ist es auch zweckmäßig, Kombinationen der einzelnen Verfahren zu benutzen. Besonders geeignet ist die Trennung durch Kristallisation oder Craig-Verteilung bzw. eine Kombination beider Verfahren.

Die enantiomerenreinen Verbindungen sind auch zugänglich durch Chromatographie der racemischen Ester auf chiralen Phasen.

Die Verbindungen der allgemeinen Formel (II) können beispielsweise hergestellt werden, indem man,
Verbindungen der allgemeinen Formel (III) 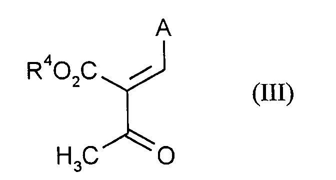
in welcher
E und R⁴ die oben angegebene Bedeutung haben,
mit Verbindungen der allgemeinen Formel (IV) 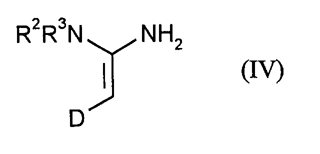
in welcher
R², R³ und D die oben angegebene Bedeutung haben,
in einem der oben aufgeführten organischen Lösemitteln, bevorzugt in Ethanol und gegebenenfalls in Anwesenheit einer Base umsetzt.

Als Basen eignen sich im allgemeinen Alkalihydride oder -alkoholate, wie beispielsweise Natriumhydrid oder Kalium-tert.butylat, oder cyclische Amine, wie beispielsweise Piperidin, Dimethylaminopyridin oder C₁-C₄-Alkylamine, wie beispielsweise Triethylamin. Bevorzugt sind Piperidin, Dimethylaminopyridin, Pyridin, Natriumhydrid und Kalium-tert.butylat.

Die Base wird im allgemeinen in einer Menge von 1 mol bis 5 mol, bevorzugt von 1 mol bis 2 mol, bezogen auf 1 mol der Verbindungen der allgemeinen Formel (III) eingesetzt.

Die Umsetzungen können bei Normaldruck, aber auch bei erhöhtem oder erniedrigtem Druck (z.B. 0,5 bis 3 bar) durchgeführt werden. Im allgemeinen arbeitet man bei Normaldruck.

Die Reaktionstemperaturen können in einem größeren Bereich variiert werden. Im allgemeinen arbeitet man zwischen +10°C und +150°C, vorzugsweise zwischen +20°C und +100°C, insbesondere bei der Siedetemperatur des jeweiligen Lösemittels.

Die Verbindungen der allgemeinen Formeln (III) und (IV) sind bekannt oder nach üblichen Methoden herstellbar.

### ⁸⁶Rubidium-Efflux aus C6-BU1-Glioma-Zellen

Die Versuche wurden mit geringfügigen Veränderungen entsprechend der von Tas et al. [Neurosci. Lett. 94, 279-284, (1988)] beschriebenen Methode durchgeführt. Dazu werden Ratten C6-BU1-Glioma-Zellkulturen verwendet. Aus den Daten wird die durch Ionomycin hervorgerufene Erhöhung des Effluxes über den Basalefflux berechnet und als 100 % gesetzt. Die Stimulationen in Gegenwart von Prüfsubstanzen werden dann auf diesen Wert bezogen.

Zur vorliegenden Erfindung gehören auch pharmazeutische Zubereitungen, die neben inerten, nicht-toxischen, pharmazeutisch geeigneten Hilfs- und Trägerstoffen eine oder mehrere Verbindungen der allgemeinen Formeln (I) / (Ia) enthalten, oder die aus einem oder mehreren Wirkstoffen der Formeln (I) / (Ia) bestehen, sowie Verfahren zur Herstellung dieser Zubereitungen.

Die Wirkstoffe der Formeln (I) / (Ia) sollen in diesen Zubereitungen in einer Konzentration von 0,1 bis 99,5 Gew.-%, bevorzugt von 0,5 bis 95 Gew.-% der Gesamtmischung vorhanden sein.

Neben den Wirkstoffen der Formeln (I) / (Ia) können die pharmazeutischen Zubereitungen auch andere pharmazeutische Wirkstoffe enthalten.

Die oben aufgeführten pharmazeutischen Zubereitungen können in üblicher Weise nach bekannten Methoden hergestellt werden, beispielsweise mit dem oder den Hilfs- oder Trägerstoffen.

Im allgemeinen hat es sich als vorteilhaft erwiesen, den oder die Wirkstoffe der Formeln (I) / (Ia) in Gesamtmengen von etwa 0,01 bis etwa 100 mg/kg, bevorzugt in Gesamtmengen von etwa 1 mg/kg bis 50 mg/kg Körpergewicht je 24 Stunden, gegebenenfalls in Form mehrerer Einzelgaben, zur Erzielung des gewünschten Ergebnisses zu verabreichen.

Es kann aber gegebenenfalls vorteilhaft sein, von den genannten Mengen abzuweichen, und zwar in Abhängigkeit von der Art und vom Körpergewicht des behandelten Objekts, vom individuellen Verhalten gegenüber dem Medikament, der Art und Schwere der Erkrankung, der Alt der Zubereitung und Applikation, sowie dem Zeitpunkt bzw. Intervall, zu welchem die Verabreichung erfolgt.

### Ausgangsverbindungen

### Beispiel I

6-Amino-4-(3-chlor-4-trifluormethylphenyl)-1,4-dihydro-2-methyl-5-nitronicotinsäuremethyl-ester 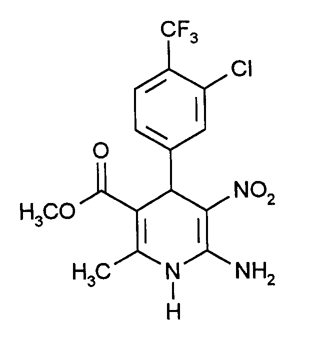
15,3 g (50 mmol) (3-Chlor-4-trifluormethylbenzyliden)acetessigsäure-methylester und 5,2 g (50 mmol) 2-Nitro-1,1-ethendiamin [Herstellung nach R. Troschütz, A. Lückel, Arch. Pharm. (Weinheim) 324, 73-77 (1991)] werden in 80 ml EtOH gelöst und 12 h unter Rückfluß gehalten. Nach Abkühlen wird der entstandene Feststoff abgesaugt und mit EtOH gewaschen. Man erhält 13,0 g (66% d. Th.) der Titelverbindung.
Smp.: 250°C

### Beispiel II

6-Acetylamino-4-(3-chlor-4-trifluormethylphenyl)-1,4-dihydro-2-methyl-5-nitronicotinsäuremethylester 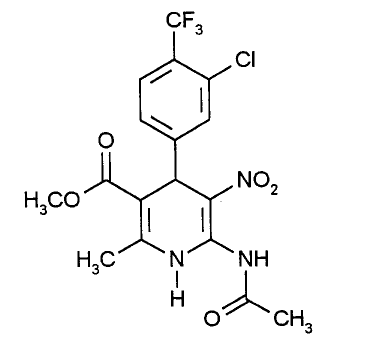
4,0 g (12,0 mmol) der Verbindung aus Beispiel I werden in 40 ml Acetanhydrid gelöst und 12 h unter Rückfluß erhitzt. Dann destilliert man das Acetanhydrid unter reduziertem Druck ab, löst den Rückstand in CH₂Cl₂ und wäscht mit gesättigter wäßriger NaHCO₃-Lösung. Die organische Phase wird getrocknet (MgSO₄), eingeengt und der Rückstand chromatographisch an Kieselgel gereinigt (Toluol/AcOEt/iPrOH 100+10+1). Das eingeengte Eluat wird aus EtOH umkristallisiert. Man erhält 0,5 g (11% d.Th.) der Titelverbindung.
Smp.: 152°C

### Beispiel III

6-Amino-5-cyan-2-methyl-4-(trifluormethylphenyl)-1,4-dihydropyridin-3-carbonsäuremethylester 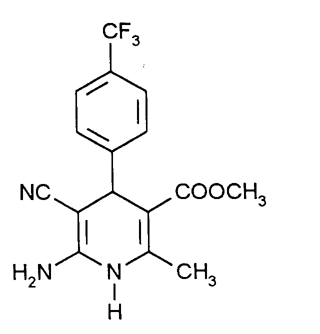
13,6 g (50 mmol) 2-Acetyl-(4-trifluormethyl)phenylessigsäuremethylester, 7,45 g (50 mmol) Cyanacetimidsäureethylesterhydrochlorid und 15 g (190 mmol) Ammoniumacetat werden in 100 ml MeOH 1 h zum Rückfluß erhitzt. Nach dem Einengen wird der Rückstand zwischen Eiswasser und AcOEt verteilt. Die organische Phase wird zweimal mit verdünnter wäßriger NaHCO₃-Lösung und einmal mit Wasser gewaschen, über Na₂CO₃ getrocknet und im Vakuum eingeengt. Kristallisation des Rückstands (18,4 g ) aus MeOH liefert 6,3 g (37% d.Th.) farblose Kristalle.
Smp.: 210-4°C

### Beispiel IV

6-Acetamido-5-cyan-4-(2,3-dichlorphenyl)-2-methyl-1,4-dihydropyridin-3-carbonsäuremethylester 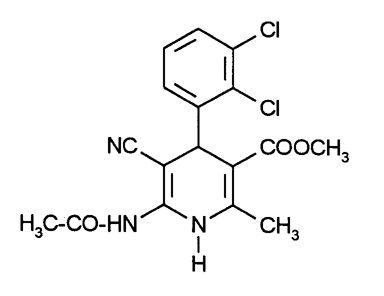
6,7 g (20 mmol) 6-Amino-5-cyan-4-(2,3-dichlorphenyl)-2-methyl-1,4-dihydropyridin-3-carbonsäuremethylester (Herstellung analog Beispiel III) und 33,5 ml (350 mmol) Acetanhydrid werden 30 min zum Rückfluß erhitzt. Überschüssiges Acetanhydrid wird anschließend durch Zugabe von MeOH unter Rühren bei 25°C zu Methylacetat umgesetzt. Die Reaktionslösung wird im Vakuum eingedampft und zweimal mit Toluol im Vakuum abgezogen. Der Rückstand wird daraufhin mit 50 ml Toluol aufgekocht. Die abgeschiedenen Kristalle werden abfiltriert und mit Toluol gewaschen.
Ausbeute: 3,6 g (50% d.Th.)
Smp.: 224°C (Zers.)

### Beispiel V

5-Cyan-4-(2,3-dichlorphenyl)-2-methyl-6-N-methylamino-1,4-dihydropyridin-3-carbonsäuremethylester 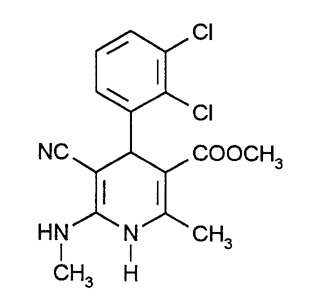
2,8 g (10 mmol) 2-Acetyl-(2,3-dichlorphenyl)essigsäuremethylester und 1,5 g (10 mol) Cyanacetimidsäureethylester-hydrochlorid werden mit 5 ml (33%iger) ethanolischer Methylaminlösung (40 mmol) versetzt. Das Gemisch erwärmt sich auf 46°C. Nach Abkühlen auf 30°C werden 2,3 ml (40 mmol) Eisessig und anschließend 20 ml MeOH zugegeben. Nach 5h Erhitzen zum Rückfluß wird die Reaktionslösung mit Eiswasser versetzt und mit AcOEt extrahiert. Trocknen der organischen Phase (Na₂SO₄) und Einengen unter reduziertem Druck liefert 3,9 g amorphen Rückstand, der an 100 g Kieselgel mit Toluol/AcOEt (Gradient) chromatographiert wird.
Ausbeute: 0,5 g (10% d.Th.) Kristalle
Smp.: 234-239°C

### Herstellungsbeispiele

### Beispiel 1

6-Amino-2-methyl-5-nitro-4-(4-trifluormethylphenyl)-nicotinsäuremethylester 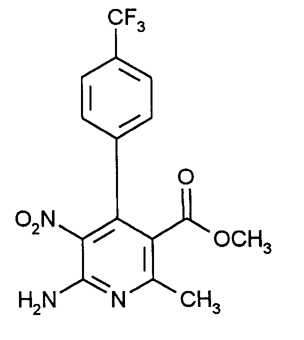
2,0 g (5,6 mmol) 6-Amino-2-methyl-5-nitro-4-(4-trifluormethyl)-1,4-dihydro-nicotinsäuremethylester (Herstellung analog Beispiel I) werden in 100 ml Methylenchlorid gelöst und mit 10,0 g Mangandioxid (gefällt, aktiv) versetzt. Man rührt 12 h bei Raumtemperatur. Dann wird der Ansatz über Kieselgel filtriert (Methylenchlorid). Das Filtrat wird eingeengt und der Rückstand aus Methanol umkristallisiert. Man erhält 1,4 g (70% d.Th.) der Titelverbindung.
Smp.: 185-186°C
In Analogie zur Vorschrift des Beispiels 1 werden die in Tabelle 1 aufgeführten Verbindungen hergestellt:

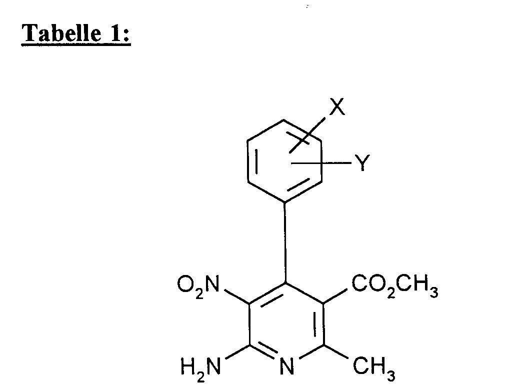

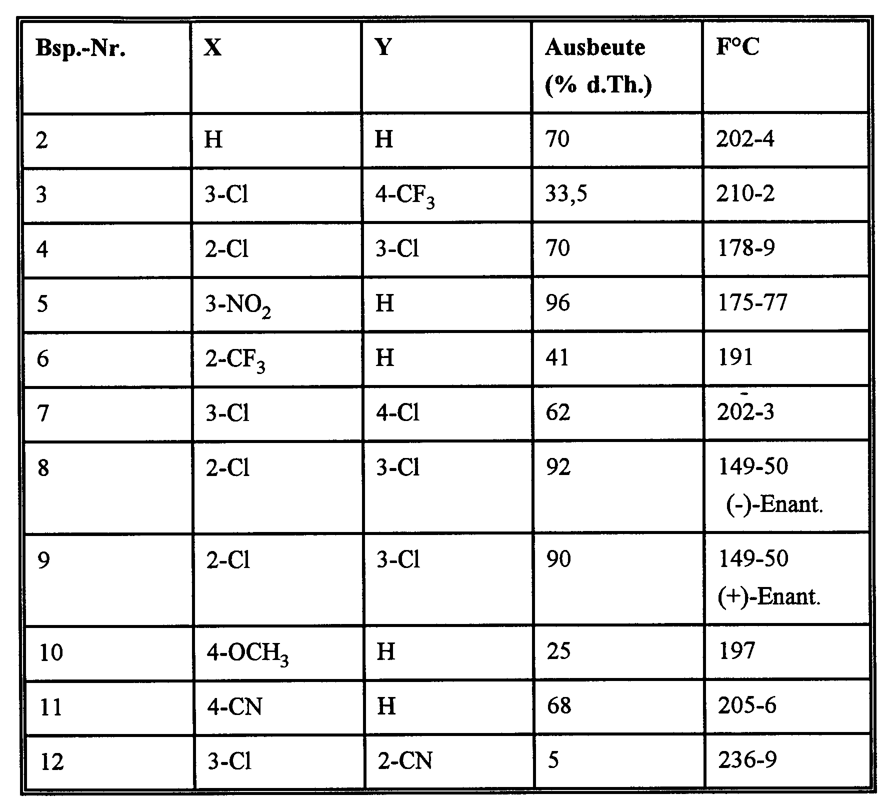

### Beispiel 13

6-N,N-Diacetylamino-2-methyl-5-nitro-4-(2-trifluormethylphenyl)-nicotinsäuremethylester 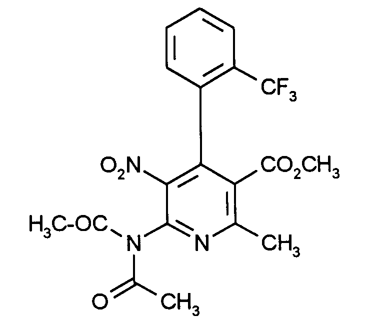
0,8 g (2,25 mmol) der Verbindung aus Beispiel 6 werden in 20 ml Acetanhydrid gelöst und über Nacht zum Rückfluß erhitzt. Nach Abdestillieren des Acetanhydrids wird der kristalline Rückstand mit Wasser gewaschen und aus Ethanol umkristallisiert. Man erhält 250 mg (28% d.Th.) der Titelverbindung
Smp.: 113°C

### Beispiel 14

4-(2,3-Dichlorphenyl)-2-methyl-6-N-methylamino-5-nitro-nicotinsäuremethylester 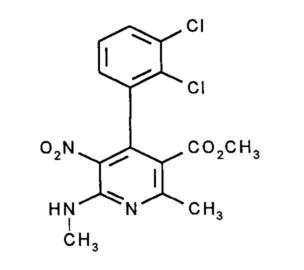
0,7 g (2 mol) der Verbindung aus Beispiel 4 werden in 20 ml trockenem DMF gelöst und mit 70 mg (80%iger) NaH versetzt. Nach 3 h Rühren bei RT versetzt man mit 2,5 ml einer 1 M Lösung aus Methyliodid in DMF und rührt erneut 18 h bei Raumtemperatur. Nach Einengen und Filtration über Kieselgel (AcOEt:Toluol 1+10) erhält man 0,8 g Rohprodukt, das säulenchromatographisch (AcOEt:Toluol 1+80) gereinigt wird. Man erhält 65 mg (9%).

In Analogie zur Vorschrift des Beispiels 14 werden die in Tabelle 2 aufgeführten Verbindungen hergestellt:

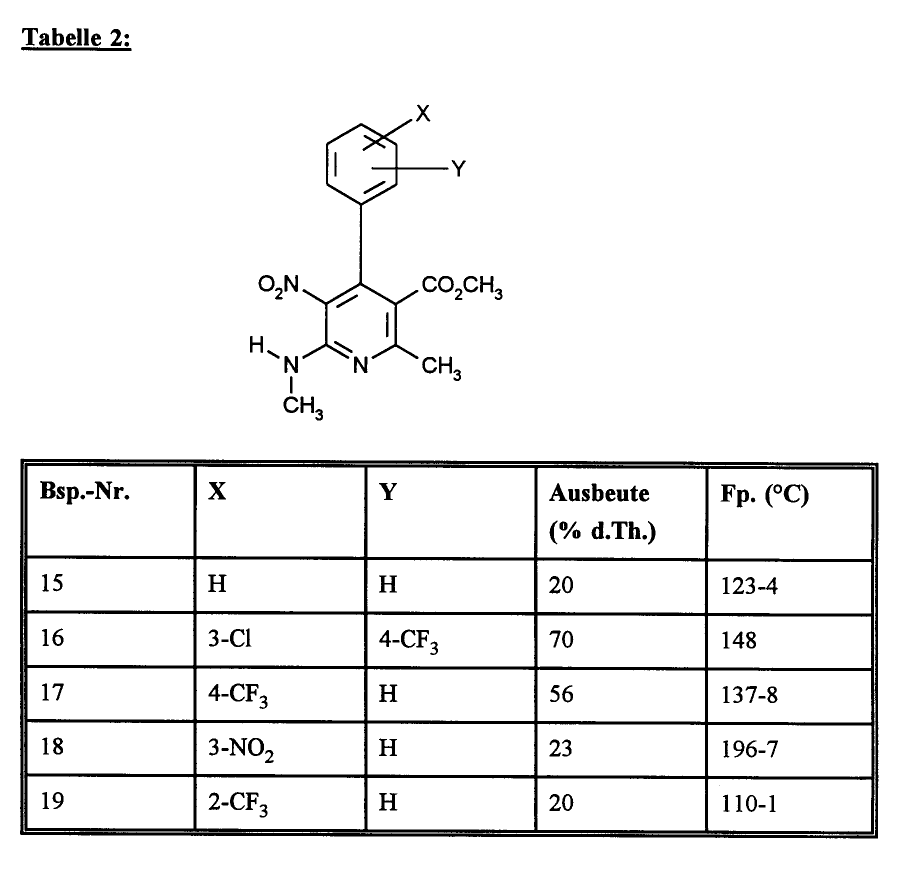

### Beispiel 20

6-N-Acetylamino-4-(2,3-dichlorphenyl)-2-methyl-5-nitronicotinsäuremethylester 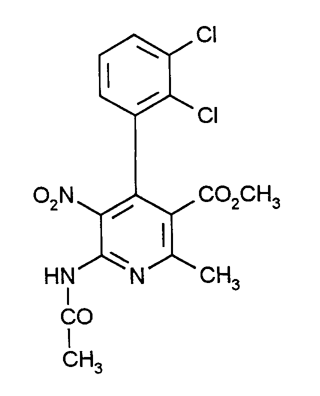
800 mg (2 mmol) 6-N-Acetylamino-4-(2,3-dichlorphenyl)-1,4-dihydro-2-methyl-5-nitronicotinsäuremethylester (Herstellung analog Beispiel II) werden in 80 ml Methylenchlorid gelöst und mit 5 g MnO₂ versetzt. Man rührt 3 d bei Raumtemperatur. Nach Filtration über Celite und Säulenchromatographie (AcOEt:Toluol 4+1) wird das eingeengte Eluat aus Toluol umkristallisiert. Man erhält 302 mg (38% d.Th.) der Titelverbindung.
Smp.: 195°C
In Analogie zur Vorschrift des Beispiels 20 werden die in Tabelle 3 aufgeführten Verbindungen herstellt:

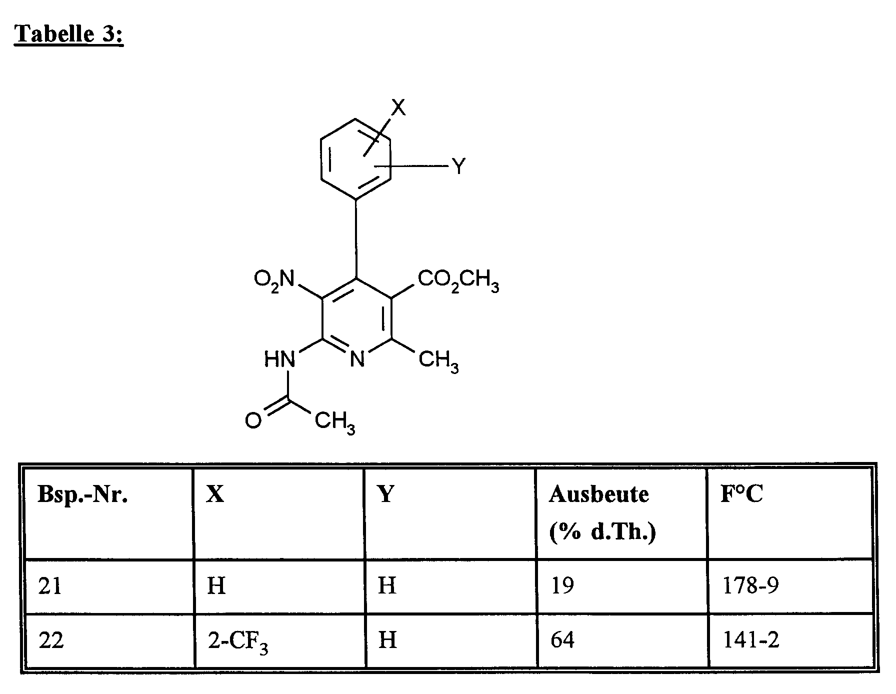

### Beispiel 23

6-Amino-5-cyano-4-(2,3-dichlorphenyl)-2-methylnicotinsäuremethylester 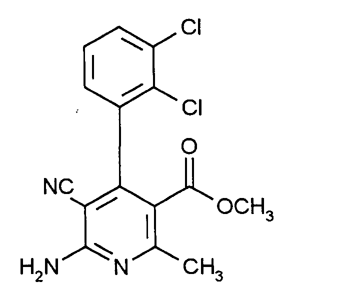
1,0 g (3 mmol) 6-Amino-5-cyano-4-(2,3-dichlorphenyl)-2-methyl-1,4-dihydro-nicotinsäuremethylester (Herstellung analog Beispiel III) und 2,6 g (30 mmol) Mangandioxid werden in 20 ml Methylenchlorid 70 h bei Raumtemperatur gerührt. Das Lösemittel wird im Vakuum abgezogen und der Rückstand mit Essigester versetzt und über Kieselgel filtriert. Man erhält 0,6 g (60% d.Th.) der Titelverindung (farblose Kristalle).
Smp.: 251-253°C

### Beispiel 24

6-Amino-5-cyan-4-(3-nitrophenyl)-2-methylnicotinsäuremethylester 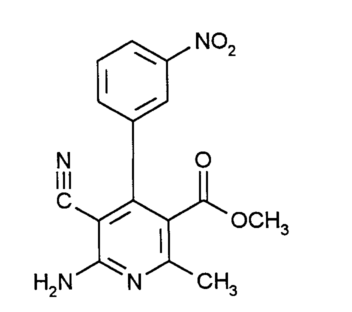
Die Titelverbindung wird in Analogie zur Vorschrift des Beispiels 20 hergestellt.
R_{f}: 0,19 (Tol : AcOC₂H₅ 3:1)
F°C: 202-5
Ausbeute: 80% d.Th.

In Analogie zur Vorschrift der Beispiele 23 und 24 werden die in Tabelle 4 aufgeführten Verbindungen hergestellt:

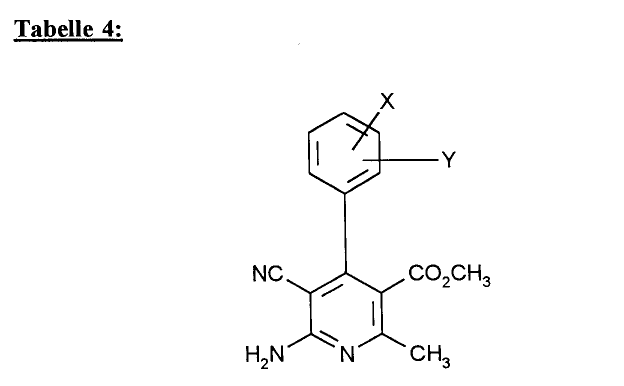

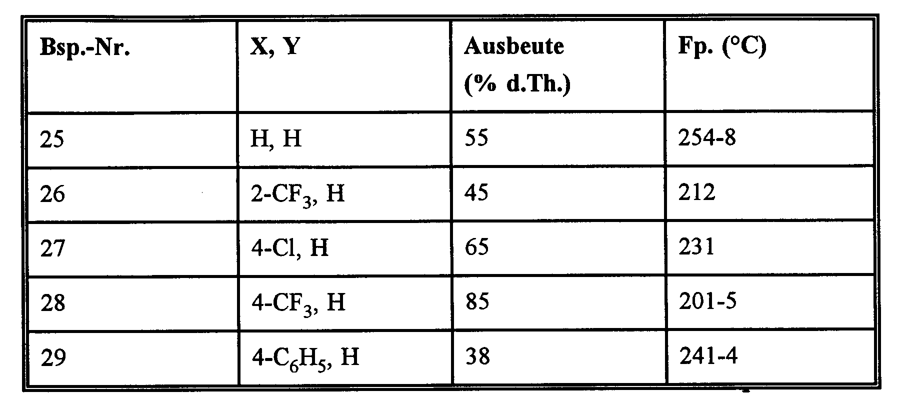

### Beispiel 30

6-Acetamido-5-cyan-4-(2,3-dichlorphenyl)-2-methyl-1,4-dihydropyridin-3-carbonsäuremethylester 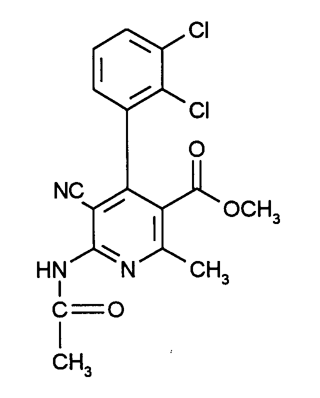
Analog zur Herstellungsvorschrift des Beispiels 23 werden 4,3 g (11 mmol) der Verbindung aus Beispiel IV durch Oxidation mit 10,7 g (120 mmol) Mangandioxid zu 1,4 g (34% d.Th.) der Titelverbindung umgesetzt.
Smp.: 160-2°C (Essigsäureethylester).

In Analogie zur Vorschrift des Beispiels 30 werden die in Tabelle 5 aufgeführten Verbindungen hergestellt:

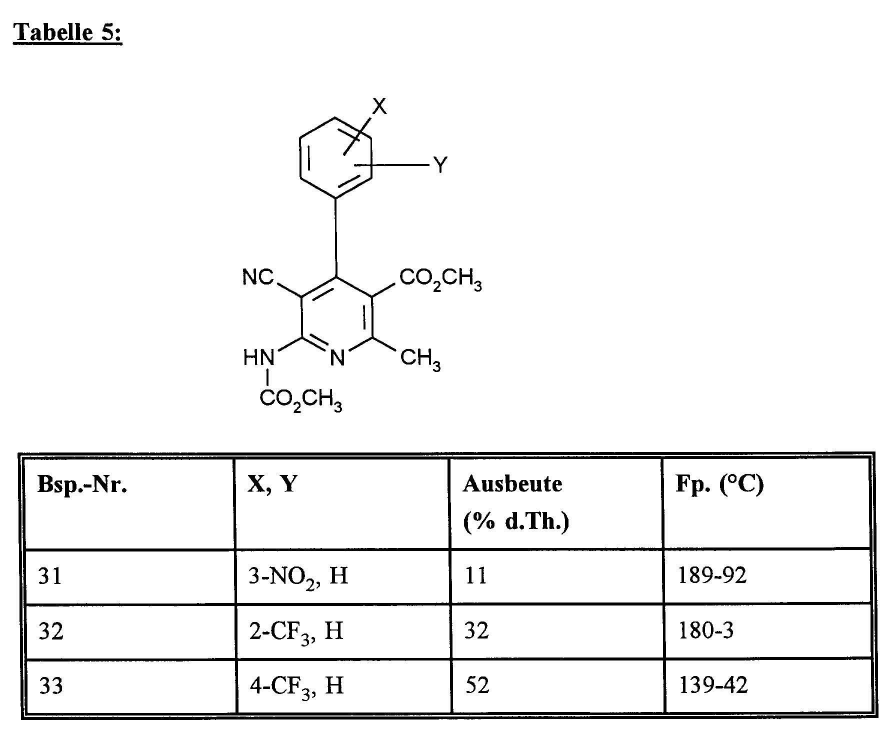

### Beispiel 34

5-Cyan-4-(2,3-dichlorphenyl)-2-methyl-6-N-methylaminopyridin-3-carbonsäuremethylester 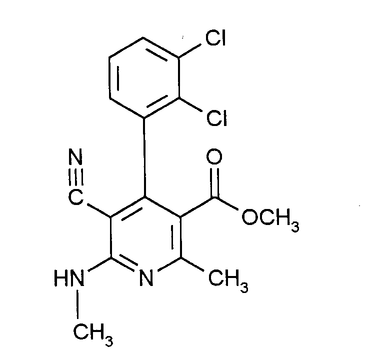
In Analogie zur Vorschrift des Beispiels 20 werden 3,5 g (10 mmol) der Verbindung aus Beispiel V durch Oxidation mit 10 g (115 mmol) Mangandioxid zu 1,7 g (49% d.Th.) der Titelverbindung umgesetzt.

In Analogie zur Vorschrift des Beispiels 34 werden die in Tabelle 6 aufgeführten Verbindungen hergestellt:

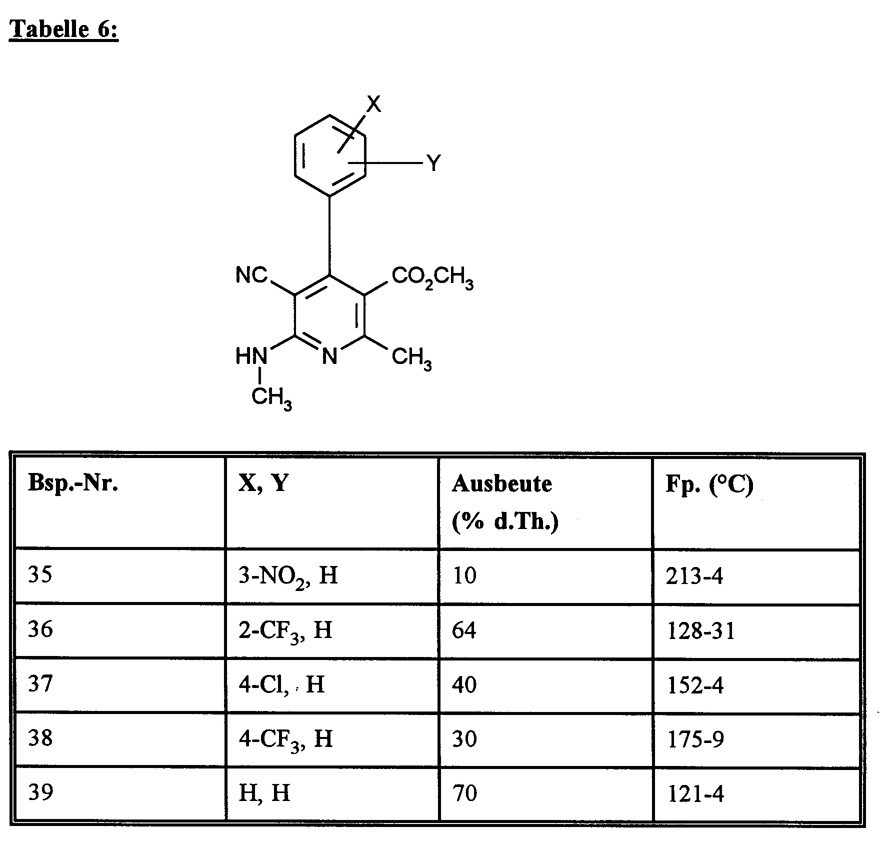

## Patentansprüche

1. Substituierte 4-Phenyl-6-amino-nicotinsäurederivate der allgemeinen Formel (I) 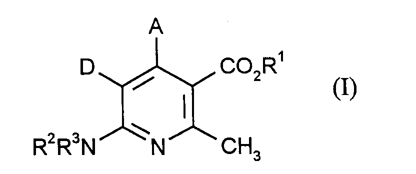 in welcher
A für Aryl mit 6 bis 10 Kohlenstoffatomen oder Pyridyl steht, die gegebenenfalls bis zu 3-fach gleich oder verschieden durch Nitro, Cyano, Phenyl, Halogen, Trifluormethyl oder durch geradkettiges oder verzweigtes Alkylthio oder Alkoxy mit jeweils bis zu 6 Kohlenstoffatomen substituiert sind,
D für Cyano oder Nitro steht,
R¹ für Wasserstoff oder für geradkettiges oder verzweigtes Alkyl mit bis zu 8 Kohlenstoffatomen steht,
R² und R³ gleich oder verschieden sind und
für Wasserstoff oder für geradkettiges oder verzweigtes Alkyl oder Acyl mit jeweils bis zu 6 Kohlenstoffatomen stehen
und deren Salze,
zur therapeutischen Anwendung.

2. Substituierte 4-Phenyl-6-amino-nicotinsäurederivate der Formel (I) nach Anspruch 1, in welcher
A für Phenyl oder Naphthyl steht, die gegebenenfalls bis zu 3-fach gleich oder verschieden durch Nitro, Cyano, Fluor, Chlor, Brom, Iod, Phenyl, Trifluormethyl oder durch geradkettiges oder verzweigtes Alkylthio oder Alkoxy mit jeweils bis zu 4 Kohlenstoffatomen substituiert sind,
D für Cyano oder Nitro steht,
R¹ für Wasserstoff oder für geradkettiges oder verzweigtes Alkyl mit bis zu 6 Kohlenstoffatomen steht,
R² und R³ gleich oder verschieden sind und
für Wasserstoff oder für geradkettiges oder verzweigtes Alkyl oder Acyl mit jeweils bis zu 4 Kohlenstoffatomen stehen
und deren Salze,
zur therapeutischen Anwendung.

3. 4-Phenyl-6-amino-nicotinsäurederivate der Formel (I) nach Anspruch 1, in welcher
A für Phenyl steht, das gegebenenfalls bis zu 2-fach gleich oder verschieden durch Nitro, Cyano, Fluor, Chlor, Brom, Iod, Phenyl, Trifluormethyl, Methoxy oder Methylthio substituiert ist,
D für Cyano oder Nitro steht,
R¹ für Wasserstoff oder für geradkettiges oder verzweigtes Alkyl mit bis zu 4 Kohlenstoffatomen steht,
R² und R³ gleich oder verschieden sind und
für Wasserstoff oder für geradkettiges oder verzweigtes Alkyl oder Acyl mit jeweils bis zu 3 Kohlenstoffatomen stehen
und deren Salze
zur therapeutischen Anwendung.

4. Arzneimittel enthaltend mindestens ein 4-Phenyl-6-amino-nicotinsäurederivat nach Anspruch 1 bis 3 sowie übliche Formulierungshilfsmittel.

5. Arzneimittel nach Anspruch 4 zur Behandlung von cerebralen Erkrankungen.

6. 4-Phenyl-6-amino-nicotinsäurederivate der allgemeinen Formel (I) 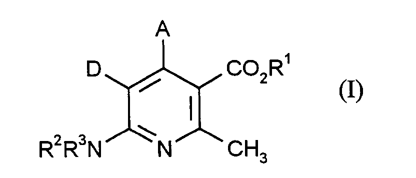 in welcher
A für Aryl mit 6 bis 10 Kohlenstoffatomen oder Pyridyl steht, die gegebenenfalls bis zu 3-fach gleich oder verschieden durch Nitro, Cyano, Phenyl, Halogen, Trifluormethyl oder durch geradkettiges oder verzweigtes Alkylthio oder Alkoxy mit jeweils bis zu 6 Kohlenstoffatomen substituiert sind,
D für Cyano oder Nitro steht,
R¹ für Wasserstoff oder für geradkettiges oder verzweigtes Alkyl mit bis zu 8 Kohlenstoffatomen steht,
R² und R³ gleich oder verschieden sind und
für Wasserstoff oder für geradkettiges oder verzweigtes Alkyl oder Acyl mit jeweils bis zu 6 Kohlenstoffatomen stehen
und deren Salze,
wobei
6-Amino-5-cyano-2-methyl-4-phenyl-nicotinsäureethylester,
6-Amino-4-(4-chlorphenyl)-5-cyano-2-methyl-nicotinsäureethylester,
6-Amino-5-cyano-2-methyl-4-(4-methoxyphenyl)-nicotinsäureethylester,
6-Amino-5-cyano-2-methyl-4-(4-nitrophenyl)-nicotinsäureethylester
ausgenommen sind.

7. Verfahren zur Herstellung von 4-Phenyl-6-amino-nicotinsäurederivaten nach Anspruch 6, dadurch gekennzeichnet, daß man
Dihydropyridine der allgemeinen Formel (II) 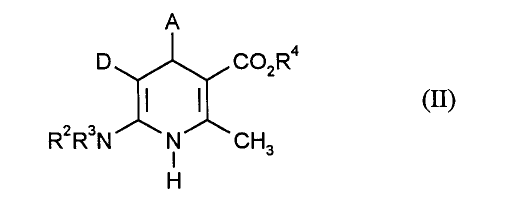 in welcher
A, D, R¹ bis R³ die angegebene Bedeutung haben,
R⁴ die oben angegebene Bedeutung von R¹ hat, aber nicht für Wasserstoff steht,
in einem inerten Lösemittel mit einem typischen Oxidationsmittel, vorzugsweise Mangandioxid, oxidiert,
die Produkte gegebenenfalls in organischen Lösemitteln und in Anwesenheit einer Base alkyliert oder acyliert
und gegebenenfalls die Ester hydrolysiert.

8. Verwendung von 4-Phenyl-6-amino-nicotinsäurederivaten nach Anspruch 1 bis 3 zur Herstellung von Arzneimitteln.

9. Verwendung von 4-Phenyl-6-amino-nicotinsäurederivaten nach Anspruch 1 bis 3 zur Herstellung von Arzneimitteln zur Behandlung von cerebralen Erkrankungen.
